# NEUE EUROPÄISCHE PATENTSCHRIFT

(11) **EP 0 971 689 B2**
(45) Veröffentlichungstag und Bekanntmachung des Hinweises auf die Entscheidung über den Einspruch: **23.03.2005**
(45) Hinweis auf die Patenterteilung: 21.08.2002
(21) Anmeldenummer: 98919190.3
(22) Anmeldetag: 30.03.1998
(51) Int. Cl.: A61K 7/50, A61K 7/06

(54) **WÄSSRIGE PFLEGENDE HAARBEHANDLUNGSMITTEL**
AQUEOUS HAIR CARE PRODUCTS
PRODUITS AQUEUX DE SOINS DES CHEVEUX

(30) Priorität: 08.04.1997 DE 19714370
(43) Veröffentlichungstag der Anmeldung: 19.01.2000
(73) Patentinhaber: Henkel Kommanditgesellschaft auf Aktien, 40589 Düsseldorf-Holthausen (DE)
(72) Erfinder: HÖFFKES, Horst, D-40595 Düsseldorf (DE); KNÜBEL, Georg, D-40229 Düsseldorf (DE); WALDMANN-LAUE, Marianne, D-40789 Monheim (DE)
(86) Internationale Anmeldenummer: PCT/EP1998/001850
(87) Internationale Veröffentlichungsnummer: WO 1998/044907

(56) Entgegenhaltungen:
- EP-A- 0 555 690
- DE-A- 3 348 135
- DE-A- 3 707 014
- DE-A- 4 009 096
- DE-A- 4 016 177
- DE-A- 4 018 259
- DE-A- 4 137 954
- DE-A- 4 333 370
- DE-C- 3 302 817
- GB-A- 716 950
- GB-A- 2 094 335
- US-A- 3 247 121
- Grundlagen und Rezepturen der Kosmetik, S. 684-5, 688-9
- Roempp Chemielexikon,S. 2460-1 (1995)

## Beschreibung

Die Erfindung betrifft wäßrige pflegende Haarbehandlungsmlttel, die als Pflegewirkstoffe eine Mischung aus Sulfatierungs- und/oder Sulfonlerungsprodukten von Carbonsäuren und/oder Hydroxycarbonsäuren sowie Fettalkoholen enthalten sowie die Verwendung derartiger Mischungen.

Tensidhaltige Zubereitungen, die zur Reinigung und Pflege der Haut oder der Haare verwendet werden, weisen häufig den Nachteil auf, daß sie zu einer starken Entfettung der Haut und/oder zu einer unerwünschten Quellung der Keratinschicht führen. Die Haut wird dadurch in einen trockenen, spröden oder rauhen Zustand versetzt, was ein unangenehmes Hautgefühl verursacht. Auch die Haare können in ihrer Struktur negativ beeinflußt werden, vor allem, wenn Mittel zur Färbung oder Wellung der Haare verwendet werden, die oxidative Wirkstoffe wie Wasserstoffperoxid oder reduktive Wirkstoffe wie Thioglykolsäure enthalten. Dies führt zu einer verschlechterten Naßkämmbarkeit oder auch zu einem nicht zufriedenstellender Griff. Den Tenside enthaltenden Haarbehandlungsmitteln werden daher Pflegekomponenten, meist quaternierte Polymere, zugesetzt. Der Einsatz dieser quaternierten Polymeren kann aber noch nicht voll zufriedenstellen, da das Haar, insbesondere bei trockener Luft, eine ungünstige elektrostatische Aufladung beim Frisieren zeigen kann. Außerdem neigen bei längerer Lagerung die Formulierungen mit quaternierten Polymeren zum Verdicken, was vor allem bei Haarfärbecremes als störend empfunden wird. Daher wurden auch Ölsäureseifen In Mischung mit Fettalkoholen als Pfiegekomponenten In Haarfärbemittel vorgeschlagen. Aber auch diese Mischung Ist nicht voll zufriedenstellend, da die Seifen mit hartem Wasser unerwünschte unlösliche Kalkselfen bilden und zudem hohe Mengen an niederen Alkoholen zur Verbesserung der rheologischen Eigenschaften notwendig sind, die aber im Hinblick auf die Hautverträglichkeit unerwünscht sind. Im Rahmen der vorliegenden Erfindung sollten die oben geschliderten Nachteile überwunden werden.

Aus der deutschen Offenlegungsschrift DE-A1-37 07 014 sind tensidhaltige Zubereitungen bekannt, deren hautkosmetische Eigenschaften merklich verbessert werden, indem man ihnen alpha-Sulfofettsäuresalze zusetzt. Für die Formulierung als kosmetische Haut- und Haarreinigungsmittel werden in der genannten Offenlegungsschrift als fakultativ enthaltene Öl- und Fettstoffe unter anderem auch Fettalkohole aufgeführt. Eine konkrete Rezeptur oder gar ein Hinweis auf eine synergistische Pflegewirkung einer Kombination aus alpha-Sulfofettsäuren und Fettalkoholen ist der Druckschrift aber nicht zu entnehmen..

Es wurde nun überraschenderweise gefunden, daß eine Mischung von Sulfatierungsund/oder Sulfonierungsprodukten von Carbonsäuren und/oder Hydroxycarbonsäuren mit Fettalkoholen eine synergistische Wirkung hinsichtlich ihrer pflegenden Eigenschaften für Haar zeigt, die weit über eine rein additive Pflegewirkung der einzelnen Mischungsbestandteile hinausgeht.

Gegenstand der vorliegenden Erfindung sind daher wäßrige pflegende Haarbehandlungsmittel enthaltend als Pflegewirkstoffe eine Mischung aus
a. Sulfatierungs- und/oder Sulfonierungsprodukten von gesättigten und/oder ungesättigten Carbonsäuren und/oder Hydroxycarbonsäuren und
b. Fettalkoholen
wobei in einen ersten Ausführungsform den pH-Wert für Shampoos, kuren und Spülungen unter 4 liegt und bei direktzeihenden Farbstoffe oder Vorprodukte für Oxidationsfarbstoffe enthaltenden Mitteln im Bereich von 6 bis 11 liegt und in einen zweiten Ausführungsform als zusätzlichen pflegenden Bestandteil Wasserlösliche Stärke und/oder Acyllactylat enthalten ist.

Bevorzugt Im Sinne der Erfindung sind Sulfonlerungsprodukte von ungesättigten und/oder gesättigten Carbonsäuren, die im Sinne der Erfindung auch kurz "Sulfonate von gesättigten oder ungesättigten Carbonsäuren" genannt werden. Häufig ist in der Literatur für die Sulfonate der gesättigten Carbonsäuren auch der Begriff "alpha-Sulfocarbonsäuran" üblich.

Sulfonate von ungesättigten Carbonsäuren sind literaturbekannt. Sie sind beispielsweise durch Umsetzung der ungesättigten Carbonsäuren mit gasförmigem Schwefeltrioxid zugänglich. Am Beispiel der Ölsäure können Einzelheiten des Herstellungsverfahrens der deutschen Offenlegungsschrift DE-A1 39 26 344, auf die ausdrücklich Bezug genommen wird, entnommen werden.

Einfach ungesättigte Carbonsäuren werden üblicherweise mit etwa der gleichen Stoffmenge an Schwefeltrioxid umgesetzt. Mehrfach ungesättigte Carbonsäuren können sowohl mit der gleichen Stoffmenge an Schwefeltrioxid als auch mit einem Überschuß an Schwefeltrioxid umgesetzt werden. Insbesondere im letzteren Fall entsteht so ein Gemisch aus einfach und mehrfach sulfonlerten Produkten. Aus Gründen der Produktqualität ist es dabei vorteilhaft, das Verhältnis der eingesetzten Stoffmengen an Schwefeltrioxid und mehrfach ungesättigter Carbonsäure auf Werte kleiner 2 zu begrenzen.

Es ist dem Fachmann bekannt, daß bei der Sulfonierung olefinisch ungesättigter Verbindungen keine reinen Verbindungen sondern Gemische erhalten werden. Bei der Sulfonierung ungesättigter Carbonsäuren werden neben den ungesättigten Carbonsäuresulfonaten die entsprechenden gesättigten Hydroxycarbonsäuresulfonate gebildet. Die beiden Produkte entstehen üblicherweise im Verhältnis von etwa 70:30 bis 30:70.

Da die Eigenschaften der ungesättigten Carbonsäuresulfonate und der gesättigten Hydroxycarbonsäuresulfonate sehr ähnlich sind, können in den erfindungsgemäßen pflegenden Mischungen anstelle der reinen ungesättigten Carbonsäuresulfonate auch die genannten Mischungen eingesetzt werden.

Im Rahmen dieser Anmeldung werden daher unter dem Begriff "Sulfonierungsprodukte von ungesättigten Carbonsäuren" oder unter dem Begriff "Sulfonate ungesättigter Carbonsäuren" sowohl die ungesättigten Carbonsäuresulfonate als auch die bei der Herstellung anfallenden Gemische aus ungesättigten Carbonsäuresulfonaten und gesättigten Hydroxycarbonsäure-sulfonaten verstanden.

Erfindungsgemäß können sowohl die Sulfonate reiner ungesättigter Carbonsäuren als auch die Sulfonate von Carbonsäuremischungen eingesetzt werden. Üblicherweise werden Mischungen eingesetzt, die aus natürlichen Rohstoffen wie beispielsweise Ölen erhalten werden.

Als ungesättigte Carbonsäuren können beispielsweise Lauroleinsäure, Myristoleinsäure, Palmitoleinsäure, Petroselinsäure. Petroselaidinsäure, Ölsäure, Elaidinsäure, Linolsäure, Linolaidinsäure, Linolensäure, Eläostearinsäure, Gadoleinsäure, Arachidonsäure, Erucasäure, Brassidinsäure, Eicosapentaensäure, Clupanodonsäure und Docosahexaensäure eingesetzt werden.

Bevorzugte ungesättigte Carbonsäuren enthalten 12 bis 24 Kohlenstoffatome und 1 bis 3 C=C-Doppelbindungen, wie beispielsweise Petroselinsäure, Ölsäure, Elaidinsäure, Linolsäure, Linolensäure, Arachidonsäure, Erucasäure und Brassidinsäure.

Besonders bevorzugt ist die Ölsäure.

Es hat sich als vorteilhaft erwiesen, derartige Sulfonierungsprodukte der ungesättigten Carbonsäuren in Form ihrer Salze, insbesondere in Form ihrer Alkalisalze, einzusetzen.

Im Sinne der Erfindung werden besonders gute pflegende Eigenschaften der Pflegemischung erhalten, wenn Sulfonierungsprodukte von gesättigten Carbonsäuren gemäß Formel (I), 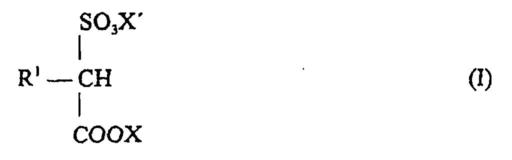
- in der R¹ =: C₆-C₂₀-Alkylgruppe,
- X,X' =: unabhängig voneinander Wasserstoff, ein Alkali-, Ammonium- oder Alkanolammonium- Kation oder eine alkalisch reagierende Aminosäure bedeuten,
eingesetzt werden.

Auch die Sulfonierungsprodukte der gesättigten Carbonsäuren sind literaturbekannt. Die Herstellung von alpha-Sulfocarbonsäuren-di-Alkali-Salzen (X, X' in Formel (I) bedeuten ein Alkali-Kation) werden beispielsweise in der deutschen Offenlegungsschrift DE-A1 39 13 385 beschrieben. Auf analoge Weise lassen sich die anderen alpha-Sulfocarbonsäuresalze der Formel (I) herstellen, wenn man zur Neutralisation die entsprechenden Basen oder alkalisch reagierenden Aminosäuren verwendet. Geeignete alkalisch reagierende Aminosäuren sind insbesondere Arginin, Histidin, Lysin, und/oder Ornithin. Geeignete Alkanolamine sind zum Beispiel Mono-, Di-, Triethanolamin. Auch die Herstellung gemischter Salze wie Natrium-Triethanolammonium-Mischsalze ist möglich, wenn man die Neutralisation mit 1 Mol Natriumhydroxid und 1 Mol Triethanolamin pro Mol alpha-Sulfocarbonsäure durchführt. Auch "saure" Salze der alpha-Sulfocarbonsäuren sind durch Neutralisation mit nur 1 Mol Base herstellbar.

Bevorzugt werden Sulfonierungsprodukte von gesättigten Carbonsäuren oder Carbonsäuremischungen ausgewählt aus der von Caprin-, Laurin-, Myristin-, Palmitin-, Stearin- und Behensäure gebildeten Gruppe. Insbesondere geeignet sind Sulfonierungsprodukte von einer Carbonsäuremischung auf Basis von Laurin- und Myristinsäure. Diese werden wiederum bevorzugt in Form ihrer Di-Salze eingesetzt.

Erfindungsgemäß bevorzugte Sulfonierungsprodukte von Hydroxycarbonsäuren sind die Sulfonierungsprodukte von C₈- bis C₂₂-Hydroxycarbonsäuren, insbesondere die Sufonierungsprodukte der Hydroxystearinsäure.

Die erfindungsgemäßen Mittel enthalten in ihrer pflegenden Mischung als weiteren zwingenden Bestandteil einen Fettalkohol.

Als Fettsäure eignen sich Insbesondere Fettsäuren mit 12 bis 22 C-Atomen und deren Mischungen. Geeignete Fettsäuren sind vor allem die gesättigten Fettsäuren, die Im Zusammenhang mit den Sulfoillerungsprodukten von gesättigten Carbonsäuren schon oben beschrieben wurden.

Besonders gute pflegende Eigenschaften Im Sinne der Erfindung zeigen Mischungen mit Fettalkoholen, Insbesondere mit 12 bis 22 C-Atomen. Geeignete Fettalkohole sind gesättigte Fettalkohole wie Capryl-, Pelargon-, Caprin-, Lauryl-, Myristyl-, Cetyl-, Stearyl-, Arachidyl- und Behenylalkohol sowie deren Mischungen.

Das Verhältnis, in dem die Komponenten a) und b), insbesondere die Sulfonierungsprodukte der gesättigten Carbonsäuren und die Fettalkohole mit 12 bis 22 C-Atomen, in dem erfindungsgemäßen Mittel vorliegen, kann in weiten Bereichen variieren. Besonders gute pflegende Eigenschaften können erzielt werden, wenn die Komponenten a) zu den Komponenten b), insbesondere die Sulfonierungsprodukte der gesättigten Carbonsäuren zu den Fettalkoholen mit 12 bis 22 C-Atomen, im Gewichtsverhältnis 20:1 bis 1:10 vorliegen.

Eine Ausführungsform der vorliegenden Erfindung betrifft wäßrige pflegende Haarbehandlungsmittel und umfaßt sowohl Mittel, die auf dem Haar verbleiben, als auch solche Mittel, die nach einer Einwirkzeit von wenigen Sekunden bis Minuten wieder ausgespült werden. Beispiele für wäßrige pflegende Haarbehandlungsmittel sind pflegende Shampoos, Spülungen, Kuren, Konditioniermittel, Tönungsmittel, Färbemittei, Dauerwellmittel, Fixiermittel und Fönwellen. In diesen wäßrigen Haarbehandlungsmittel sind die Pflegemischungen in Mengen von 5 bis 40 Gew.%, bezogen auf das wäßrige Haarbehandlungsmittel, enthalten.

Es hat sich weiterhin gezeigt, daß praktisch unabhängig von der Art des wäßrigen Haarbehandlungsmittels bestimmte weitere Komponenten die pflegende Mischung in Ihrer Wirkung besonders vorteilhaft ergänzen. Solche weiteren Komponenten sind insbesondere
- Acyllactylate und
- wasserlösliche Stärke,
die alleine oder in Mischung miteinander enthalten sein können.

Geeignete Acyllactylate haben die allgemeine Formel (II),

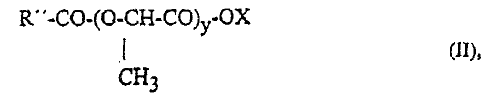

in der R" für eine lineare oder verzweigte, gesättigte oder ungesättigte Alkyl- oder Alkenylgruppe mit 6 bis 22 Kohlenstoffatomen steht, X für ein Alkali-, Ammonium- oder Alkanolammonium-Kation oder eine alkalisch reagierende Aminosäure und y für eine Zahl von 1 bis 5.

Acyllactylate, bei denen R" für eine, insbesondere gesättigte, lineare oder methylverzweigte, Alkylgruppe mit 12 bis 18 Kohlenstoffatomen steht und y für eine Zahl von 1 bis 3, sind besonders bevorzugt.

Solche Acyllactylate sind im Handel unter dem Warenzeichen Pationic® erhältlich.

Ein Natrium-isostearoyllactyllactylat, das unter der Bezeichnung Pationic®ISL vertrieben wird, ist ein besonders bevorzugtes Acyllactylat.

Schließlich haben sich auch Mittel, die zusätzlich wasserlösliche Stärke enthalten, als besonders wirksam erwiesen.

Je nach Anwendungstyp der wäßrigen Haarbehandlungsmittel können noch in dem Fachmann bekannten üblichen Mengen enthalten sein:
- nichtionische Tenside wie Fettalkoholalkoxylate und Alkylpolyglykoside
- anionische Tenside, wie beispielsweise Alkylbenzolsulfonate, Glycerinethersulfonate, Alkansulfonate, Alkylsulfate, Alkylethersulfate mit konventioneller oder eingeschränkter Homologenverteilung, Glycerinethersulfate, Monoglycerid(ether)sulfate, Hydroxymischethersulfate, Alkyloligoglucosidsulfate, Isethionate, Tauride, Sarcosinate, Alkylethercarbonsäuresalze, Sulfosuccinate, Sulfotriglyceride und Alkyl(ether)phosphate
- kationische Tenside, wie beispielsweise quartäre Ammoniumverbindungen, Amidoamine und quaternisierte Ester und Proteinhydrolysate,
- zwitterionische Tenside, wie beispielsweise Betaine,
- ampholytische Tenside,
- nichtionische Polymere wie beispielsweise Polyvinylpyrrolidon, Vinylpyrrolidon/Vinylacetat-Copolymere und Celluloseether,
- Strukturanten wie Glucose und Maleinsäure,
- haarkonditionierende Verbindungen wie Phospholipide, beispielsweise Sojalecithin, Ei-Lecithin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle und Dimethylisosorbid,
- Lösungsvermittler, wie Ethanol, Isopropanol, Ethylenglykol, Propylenglykol, Glycerin und Diethylenglykol,
- Farbstoffe,
- Antischuppenwirkstoffe wie Piroctone Olamine und Zink Omadine,
- weitere Substanzen zur Einstellung des pH-Wertes,
- Wirkstoffe wie Panthenol, Allantoin, Pyrrolidoncarbonsäuren und deren Salze, Pflanzenextrakte und Vitamine,
- Lichtschutzmittel,
- Konsistenzgeber wie Zuckerester, Polyolester oder Polyolalkylether,
- Wachse, wie Bienenwachs und Montanwachs,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien,
- Reduktionsmittel wie z.B. Thioglykolsäure und deren Derivate, Thiomilchsäure, Cysteamin, Thioäpfelsäure und α-Mercaptoethansulfonsäure,
- Oxidationsmittel wie Wasserstoffperoxid, Kaliumbromat und Natriumbromat.

Für besonders pflegende Haarbehandlungsmittel mit Tensiden empfehlen sich als besonders milde Tenside Alkylpolyglykoside gemäß der Formel RO-(Z)ₓ, in der R steht für einen Alkylrest mit 8 bis 22 Kohlenstoffatomen, Z für einen Mono- oder Oligosaccharid und x für eine Zahl von 1,1 bis 5. Diese Verbindungen sind durch die folgenden Parameter gekennzeichnet.

Der Alkylrest R enthält 8 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die geeigneten Alkylpolyglykoside enthalten im Schnitt 1,1 bis 5 Zuckereinhelten. Alkylglykoside mit x-Werten von 1,3 bis 2, insbesondere mit x-Werten von 1,4 bis 1,6, können besonders bevorzugt sein.

Werden ganz besonders pflegende wäßrige Haarbehandlungsmittel gewünscht, können neben derbeschriebenen Pflegemischung selbstverständlich die bekannten pflegenden katlonlschen Polymere wie die unter den Handelsbezeichnungen Polymer JR®, Cosmedia Guar®, Luviquat®, Mirapol® A15, Gafquat®, Merquat®, Hercules PD®170, Cartaretine®F4, Polyquart®H und Polymin®HS. Die kationischen Polymeren können in Mengen von 1 bis 10 Gew.%, bezogen auf das Haarbehandlungsmittel, enthalten sein.

Die erfindungsgemäß verwendeten Mittel können beispielsweise als wäßrige oder wäßrigalkoholische Lösungen, Cremes, Gele oder Emulsionen formuliert sein. Als Alkohole kommen die bekannten niedermolekularen Alkohole wie Ethanol und Isopropanol in Betracht.

Als besonders vorteilhaft hat sich die pflegende Mischung in Mitteln zum Färben und Tönen von Haaren erwiesen.

Solche Mittel enthalten sogenannte "direktziehende" Farbstoffe und/oder Vorprodukte für Oxidationsfarbstoffe (sogenannte Farbstoffvorprodukte).

Übliche direktziehende Farbstoffe sind z. B. Nltrobenzolderlvate, Antrachinon-Farbstoffe, Triphenylmethanfarbstoffe und Azofarbstoffe. In Haariärbe- und Tönungsmittein eingesetzte Vertreter dieser Farbstoffklasse sind beisplelswelse die unter den internationalen Bezeichnungen bzw. Handelsnamen HC Yellow 2, HC Yellow 4, Methylgelb, Fluorgelb, Basic Yellow 57, Disperse Orange 3, HC Red 3, HC Red BN, Kardinalrot, Rot Y, Rot X, Basic Red 76, HC Blue 2, Nitroblau, Disperse Blue 3, Basis Blue 99, HC Violet 1, Disperse Vlolet 1, Disperse Violet 4, Disperse Black 9, Basis Brown 16, Pikraminsäure und Rodol 9 R, bekannten Verbindungen. Weitere geeignete direktziehende Farbstoffe sind beispielsweise die Verbindungen 3-Nitro-4-ethylaminobenzoesäure, 1,2,3,4-Tetrahydro-6-nitro-chinoxalin, 2-Nitro-4-amino-4'-dimethylaminodiphenylamin-2'-carbonsäure und 2-Nitro-4-aminodiphenylamin-2'-carbonsäure.

Als Vorprodukte für Oxidationsfarbstoffe enthalten Haarfärbemlttel sogenannte Entwicklerund Kuppierkomponenten. Die Entwicklerkomponenten bilden unter dem Einfluß von Oxidationsmitteln oder Luftsauerstoff untereinander oder unter Kupplung mit einer oder mehreren Kupplerkomponenten die eigentlichen Farbstoffe aus.

Als Entwicklerkomponenten werden üblicherweise primäre aromatische Amine mit einer weiteren, in paraoder ortho-Position befindlichen freien oder substituierten Hydroxy- oder Aminogruppe, Diaminopyridinderivate, heterocycllsche Hydrazone, 4-Amlnopyrazolonderlvate sowie 2,4,5,6-Tetraaminopyrimidin und dessen Derivate eingesetzt. Spezielle Vertreter sind beispielsweise p-Toluylendlamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5 und 4-Amino-3-methylphenol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere α-Naphthol, Pyrogallol, 1,5-, 2,7- und 1,7-Dihydroxynaphthalin, 5-Amino-2-methylphenol, m-Aminophenol, Resorcin, Resorcinmonomethylether, m-Phenylendlamin, 1-Phenyl-3-methyl-pyrazolon-5,2,4-Dichlor-3-aminophenal, 1,3-Bis-(2,4-diaminophenoxy)-propan, 2-Chlor-resorcin, 2-Chlor-6-methyl-3-aminophenol und 2-Methylresorcin.

Die pflegenden Eigenschaften der erfindungsgemäßen wäßrigen Haarbehandlungsmittel kommen besonders dann zur Geltung, wenn diese für Shampoos, Kuren und Spülungen so formuliert werden, daß sie einen pH-Wert unterhalb von 4 aufweisen. Die pflegenden Eigenschaften für wäßrige pflegende Haarbehandlungsmittel, die direktziehende Farbstoffe oder Vorprodukte für Oxidationsfarbstoffe enthalten, kommen besonders dann gut zur Geltung, wenn diese einen pH-Wert im Bereich von 6 bis 11 aufweisen, insbesondere von 8 - 10.

Besonders bevorzugt ist die Verwendung einer Mischung aus Suifonierungsprodukten von gesättigten Carbonsäuren gemäß Formel (I) und Fettalkoholen mit 12 bis 22 C-Atomen. Einzelhelten zu den Verbindungen der erfindungsgemäß verwendeten Mischung als auch zu weiteren fakultativen Bestandteilen sind bereits oben beschrieben worden.

Besonders geeignet ist die erfindungsgemäß verwendete Pflegewirkstoffmischung für direktziehende oder Vorprodukte für Oxidationsfarbstoffe enthaltende Haarfärbe- und Haartönungsmittel.

### Beispieie

### B. Haarbehandlungsmittel

Die Bestimmung der Pflegewirkung wurde an vorgeschädigten Haaren vorgenommen. Zur gezielten Schädigung der Haare wurden trockene Haarsträhnen von ca. 2 g Gewicht (Fischbach und Miller Typ6923) einmal mit 32 g Blondlermlschung (Marktprodukt Poly Blond Medium Aufheller) eine halbe Stunde lang blondiert. Nach dem Auswaschen der Biondiermischung wurden die Haarsträhnen unmittelbar ohne Zwischentrocknung einer Dauerwellbehandlung mit dem Marktprodukt Poly Lock Normal unterzogen. Die Elnwirkdauem von Wellkomponente und Fixierkomponente betrugen dabei 30 bzw. 15 Minuten. Nach dem Ausspülen der Fixierkomponente wurden die Strähnen getrocknet und mindestens zwei Tage bei Umgebungsbedingungen konditioniert.

Die gezieitvorgeschädigten Haare wurden anschließend hinsichtlich der Naßkämmbarkeit geprüft. Dazu wurde die gespülte Haarsträhne vor der Prüfung mit je 0,2 ml einer 50%igen Lösung von Texapon® N25 (28%ige Lösung von Natriumlaurylethersulfat in Wasser) intensiv shampooniert und anschließend ausgespült. Danach wurden 0,5 ml der zu prüfenden Formulierung gleichmäßig in das Haar massiert, eine Minute im Haar belassen und dann sorgfältig ausgespült. Danach wurde mit einem feinzinkigen Kamm gekämmt und der Kämmwiderstand subjektiv beurteilt. Anschließend wurde an derselben Strähne in gleicher Weise die zu vergleichende Formulierung geprüft. Die Beurteilung wurde entsprechend einem Beurteilungssystem von 1 (= sehr gut) bis 6 (= ungenügend) abgegeben.

### 1. Wäßrige Haarkur

Es wurden eine erfindungsgemäße wäßrige Haarkur (E2) und zwei als Vergleichsbeispiel dienende Haarkuren (V2/V3) hergestellt aus folgenden Bestandteilen (Angaben in Gew.%, bezogen auf die Haarkur):

| | E2 | V2 | V3 |
|---|---|---|---|
| Laurin-/Myristinsäuresulfonat (Aktivsubstanzgehalt 25,5 %) | 20 | 20 | -- |
| Eumulgin® B 2 ³ | -- | -- | 20 |
| Stenol® 1618 | 5 | - | 5 |
| Pationic® ISL ⁴ | 3 | 3 | 3 |
| Parfümöl | 0,3 | 0,3 | 0,3 |
| Konservierungsmittel | 0,2 | 0,2 | 0,2 |
| Zitronensäure ad pH = 2 | | | |
| Wasser ad | 100 | 100 | 100 |

| | | | |
|---|---|---|---|
| ³ Cetylstearylalkohol, ethoxyliert mit 20 Mol EO; Handelsprodukt der Henkel KGaA | | | |
| ⁴ Natriumsalz des Isostearoyllactyllactylates (INCI-Bezeichnung: Sodium Isostearoyl-2-lactate), Handelsprodukt der PATCO | | | |

Die erfindungsgemäße wäßrige Haarkur (E2) erhlelt In der Naßkämmbarkeit die Note 1, die Haarkuren V2 und V3erhielten die Noten 4-5 (V2) beziehungsweise 4 (V3). Außerdem verlieh die erfindungsgemäße Haarkur (E2) dem Haar Im Praxistest einen verbesserten Frisurenhalt.

### 2. Wäßrige Haarspülung

Es wurde eine erfindungsgemäße wäßrige Haarspülung (E3) und eine als Vergleichsbeispiel dienende Haarspülung (V4) hergestellt aus folgenden Bestandteilen (Angaben in Gew.%, bezogen auf die Spülung):

| | E3 | V4 |
|---|---|---|
| Ölsäuresulfonat-mono-K-Salz (50% Aktivsubstanzgehalt) | 10 | 10 |
| Stenol® 1618 | 5 | - |
| Stärke⁵ | 3 | 3 |
| Parfümöl | 0,3 | 0,3 |
| Konservierungsmittel | 0,2 | 0,2 |
| Wasser ad | 100 | 100 |

| | | |
|---|---|---|
| ⁵ löslich, reinst, Handelsprodukt der Firma Merck | | |

Die Naßkämmbarkeit wurde wie oben beschrieben an dem gezielt vorgeschädigten Haaren wie oben beschrieben geprüft. Die erfindungsgemäße Haarspülung (E3) erhielt die Note 3, die Vergleichsspülung (V4) die Note 4-5.

### 3. Haartönungsmittel

Es wurde ein erfindungsgemäßes Haartönungsmittel (E4) sowie ein als Vergleichsbeispiel dienendes Haartönungsmittel (V5) aus folgenden Bestandteilen hergestellt (Angaben in Gew.%,bezogen auf das Haartönungsmittel):

| | E4 | V5 |
|---|---|---|
| Laurin-/Myristinsäuresulfonat-di-Na-Salz (Aktivsubstanzgehalt 25,5%) | 20 | -- |
| Natriumlaurylethersulfat, Texapon® N25 (Aktivsubstanzgehalt 28%) | -- | 20 |
| Stenol® 1618 | 5 | 5 |
| 4-Amino-2-nitrodiphenylamin-2'-carbonsäure | 0,1 | 0,1 |
| 6-Nitro-1,2,3,4-tetrahydrochinoxalinhydrochlorid | 0,1 | 0,1 |
| Ammoniak ad pH = 8,2 | | |
| Wasser ad | 100 | 100 |

Es wurde die Naßkämmbarkeit an gezielt vorgeschädigtem Haar geprüft. Das erfindungsgemäße Haartönungsmittel (E4) erhielt die Note 2-3, das Vergleichsmittel (V5) die Note 4-5.

### 4. Haarfärbemittel

Es wurde ein erfindungsgemäßes wäßriges Haarfärbemittel (E5) und ein zum Vergleich dienendes Haarfärbemittel (V6) aus folgenden Bestandteilen hergestellt (Angaben in Gew.%, bezogen auf das Haarfärbemittel):

| | E5 | V6 |
|---|---|---|
| Laurin-/Myristinsäuresulfonat-di-Na-Salz (Aktivsubstanzgehalt 25,5 %) | 20 | -- |
| Natriumlaurylethersulfat, Texapon® N 25 (Aktivsubstanzgehalt 28 %) | 4 | 24 |
| Plantaren® 600 ⁶ | 2 | 2 |
| Akypo® Soft 45 NV ⁷ | 3 | 3 |
| Talgfettalkohol | 8 | 8 |
| Polymer JR® 400 ⁸ | 0,5 | 0,5 |
| Kationische Emulsion DC®929 | 5 | 5 |
| p-Toluylendiaminsulfat | 0,1 | 0,1 |
| p-Aminophenolhydrochlorid | 0,03 | 0,03 |
| Resorcin | 0,04 | 0,04 |
| m-Aminophenol Ammoniak ad pH = 9,5 | 0,004 | 0,004 |
| Wasser ad | 100 | 100 |

| | | |
|---|---|---|
| ⁶ flüssige, wäßrige Zubereitung von Laurylpolyglykosid; Handelsprodukt der Henkel KGaA | | |
| ⁷ Laurylalkoholethoxylat-Essigsäure-Natriumsalz (INCI-Bezeichnung: Sodium Laureth-6 Carboxylate); Handelsprodukt der Chem-Y | | |
| ⁸ quaternierte Hydroxyethylcellulose (INCI-Bezeichnung: Polyquaternium-10); Handelsprodukt von Amerchol | | |

Ausgefärbt wurde 30 Minuten bei Raumtemperatur mit einer kommerziellen Wasserstoffperoxiddispersion der Poly Color Brillance Color-Creme im Mischungsverhältnis 1:1 unter Verwendung von Haarsträhnen der Firma Kerling mit der Bezeichnung "naturweiß". Es resultierte eine hellblonde Nuance. Die Naßkämmbarkeit wurde wie oben beschrieben ermittelt. Für das erfindungsgemäße Haarfärbemittel (E5) wurde die Note 2 erzielt, beim Vergleichsmittel (V6) die Note 4-5.

## Patentansprüche

1. Wässrige pflegende Haarbehandlungsmittel enthaltend als Pflegewirkstoffe eine Mischung aus
a. Sulfatierungs- und/oder Sulfonierungsprodukten von gesättigten und/oder ungesättigten Carbonsäuren und/oder Hydroxycarbonsäuren und
b. Fettalkoholen,
**dadurch gekennzeichnet, dass** der pH-Wert für Shampoos, Kuren und Spülungen unter 4 liegt und bei direktziehenden Farbstoffe oder Vorprodukte für Oxidationsfarbstoffe enthaltenden Mitteln im Bereich von 6 bis 11 liegt.

2. Wässrige pflegende Haarbehandlungsmittel enthaltend als Pflegewirkstoffe eine Mischung aus
a. Sulfatierungs- und/oder Sulfonierungsprodukten von gesättigten und/oder ungesättigten Carbonsäuren und/oder Hydroxycarbonsäuren und
c. Fettalkoholen,
**dadurch gekennzeichnet, dass** als zusätzlicher pflegender Bestandteil wasserlösliche Stärke und/oder Acyllactylat enthalten ist.

3. Wässrige pflegende Haarbehandlungsmittel nach einem der Ansprüche 1 oder 2, **dadurch gekennzeichnet, dass** Sulfonierungsprodukte von gesättigten und/oder ungesättigten Carbonsäuren und/oder Hydroxycarbonsäuren enthalten sind.

4. Wässrige pflegende Haarbehandlungsmittel nach einem der Ansprüche 1 bis 3, **dadurch gekennzeichnet, dass** Sulfonierungsprodukte von gesättigten Carbonsäuren gemäß Formel (I) 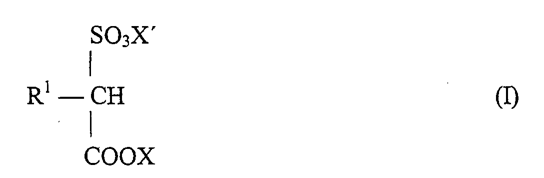
in der R¹ = C₆-C₂₀-Alkylgruppe,
X,X' = unabhängig voneinander Wasserstoff, ein Alkali-, Ammonium- oder Alkanolammonium-Kation oder eine alkalisch reagierende Aminosäure
bedeuten, enthalten sind.

5. Wässrige pflegende Haarbehandlungsmittel nach einem der Ansprüche 1 bis 4, **dadurch gekennzeichnet, dass** als Pflegewirkstoffe eine Mischung aus Sulfonierungsprodukten von gesättigten Carbonsäuren und Fettalkoholen mit 12 bis 22 C-Atomen enthalten sind.

6. Wässrige pflegende Haarbehandlungsmittel nach Anspruch 5, **dadurch gekennzeichnet, dass** die Sulfonierungsprodukte der gesättigten Carbonsäuren zu den Fettalkoholen mit 12 bis 22 C-Atomen im Gewichtsverhältnis 20:1 bis 1:10 enthalten sind.

7. Wässrige pflegende Haarbehandlungsmittel nach einem der Ansprüche 1 bis 6, **dadurch gekennzeichnet, dass** sie zusätzlich Fettsäuren enthalten.

8. Wässrige pflegende Haarbehandlungsmittel nach einem der Ansprüche 1 bis 7, **dadurch gekennzeichnet, dass** sie direktziehende Farbstoffe und/oder Vorprodukte für Oxidationsfarbstoffe enthalten.

9. Wässrige pflegende Haarbehandlungsmittel nach einem der Ansprüche 1 bis 8, **dadurch gekennzeichnet, dass** ein kationisches Polymeres enthalten ist.

## Claims

1. Water-containing hair-care compositions containing as their active care ingredient a mixture of
a. sulfation and/or sulfonation products of saturated and/or unsaturated carboxylic acids and/or hydroxycarboxylic acids and
b. fatty alcohols.
**characterized in that** the pH values for shampoos, after treatments and rinses is below 4 and, for compositions containing substantive dyes or oxidation dye precursors, is in the range from 6 to 11.

2. Water-containing hair-care compositions containing as their active care ingredient a mixture of
a. sulfation and/or sulfonation products of saturated and/or unsaturated carboxylic acids and/or hydroxycarboxylic acids and
b. fatty alcohols.
**characterized in that** water-soluble starch and/or acyl lactylate is present as an additional care ingredient.

3. Water-containing hair-care compositions as claimed in any of claims 1 or 2, **characterized in that** sulfonation products of saturated and/or unsaturated carboxylic acids and/or hydroxycarboxylic acids are present.

4. Water-containing hair-care compositions as claimed in any of claims 1 to 3, **characterized in that** sulfonation products of saturated carboxylic acids corresponding to formula (I): 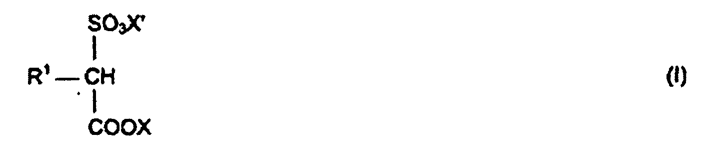 in which
R¹ is a C₆₋₂₀ alkyl group,
X and X' independently of one another represent hydrogen, an alkali metal, ammonium or alkanolammonium cation or an alkaline amino acid, are present.

5. Water-containing hair-care compositions as claimed in any of claims 1 to 4, **characterized in that** a mixture of sulfonation products of saturated carboxylic acids and C₁₂₋₂₂ fatty alcohols is present as the active care ingredient.

6. Water-containing hair-care compositions as claimed in claim 5, **characterized in that** the sulfonation products of the saturated carboxylic acids and the C₁₂₋₂₂ fatty alcohols are present in a ratio by weight of 20:1 to 1:10.

7. Water-containing hair-care compositions as claimed in any of claims 1 to 6, **characterized in that** they additionally contain fatty acids.

8. Water-containing hair-care compositions as claimed in any of claims 1 to 7, **characterized in that** they contain substantive dyes and/or oxidation dye precursors.

9. Water-containing hair-care compositions as claimed in any of claims 1 to 8, **characterized in that** a cationic polymer is present.

## Revendications

1. Produits de traitement des cheveux, aqueux, soignants, contenant comme principes actifs de soins, un mélange à base de
a) produits de sulfatation et/ou de sulfonation d'acides carboxyliques saturés et/ou non saturés et/ou d'acides hydroxycarboliques et
b) d'alcools gras.
**caractérisés en ce que** la valeur du pH pour des shampooings des traitements et des rinçages se situe en dessous de 4, et pour des produits contenant des colorants à action directe ou des produits précurseurs pour colorants par oxydation, se situe dans la zone de 6 à 11.

2. Produits de traitement des cheveux, aqueux, soignants, contenant comme principes actifs de soins, un mélange à base de
a) produits de sulfatation et/ou de sulfonation d'acides carboxyliques saturés et/ou non saturés et/ou d'acides hydroxycarboliques et
c) d'alcools gras.
**caractérisés en ce qu'**ils contiennent comme constituant supplémentaire soignant, de l'amidon soluble dans l'eau et/ou un acyllactylate.

3. Produits de traitement des cheveux, aqueux, soignants selon l'une des revendications 1 ou 2,
**caractérisés en ce que** des produits de sulfonation d'acides carboxyliques saturés et/ou non saturés et/ou d'acides hydroxycarboxyliques, sont contenus.

4. Produits de traitement des cheveux soignants, aqueux selon l'une des revendications 1 à 3,
**caractérisée en ce que** des produits de sulfonation d'acides carboxyliques saturés conformément à la formule (I) 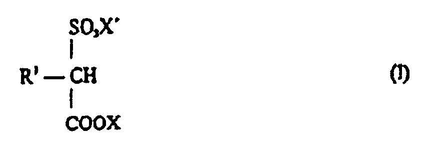 dans laquelle R¹ signifie un groupe alkyle en C₆ à C₂₀,
X et X' indépendamment l'un de l'autre signifient de l'hydrogène, un cation de métal alcalin, d'ammonium ou d'alkanol ammonium, ou un amino acide à réaction alcaline, sont contenus.

5. Produits de traitement des cheveux, aqueux, soignants, selon l'une des revendications 1 à 4,
**caractérisés en ce que** comme principe actif de soins un mélange à base de produits de sulfonation d'acides carboxyliques saturés et d'alcools gras ayant de 12 à 22 atomes de carbone, est contenu.

6. Produits de traitement des cheveux aqueux, soignants, selon la revendication 5,
**caractérisés en ce que** les produits de sulfonation des acides carboxyliques saturés sont contenus dans un rapport en poids par rapport aux alcools gras ayant de 12 à 22 atomes de carbone, allant de 20 : 1 à 1 : 10.

7. Produits de traitement des cheveux aqueux, soignants, selon l'une des revendications 1 à 6,
**caractérisés en ce qu'**ils contiennent en supplément des acides gras.

8. Produits de traitement des cheveux aqueux soignants selon l'une des revendications 1 à 7,
**caractérisés en ce qu'**ils contiennent des colorants à action directe et/ou des produits précurseurs pour des colorants par oxydation.

9. Produits de traitement des cheveux, aqueux, soignants selon l'une des revendications 1 à 8,
**caractérisés en ce qu'**ils contiennent un polymère cationique.
